# EUROPEAN PATENT APPLICATION

(11) **EP 1 612 708 A1**
(43) Date of publication of application: **04.01.2006**
(21) Application number: 04425481.1
(22) Date of filing: 30.06.2004
(51) Int. Cl.: G06F 19/00

(54) **Clinical trial phase simulation method and clinical trial phase simulator for drug trials**

(71) Applicant: BRACCO IMAGING S.p.A., 20134 Milano (IT)
(72) Inventor: Grossi, Enzo, 20161 Milano (IT); Buscema, Paolo Massimo, 00163 Roma (IT)
(74) Representative: Karaghiosoff, Giorgio Alessandro

(57) **Abstract**

A clinical trial phase simulation method for drug trials, which method allows to predict the trend of the results of a clinical trial phase of a drug with the steps of providing a database comprising for each of a certain number of individuals a predefined number of independent variables each of which corresponds to a certain clinical parameter relevant or characteristic for a disease condition against which the drug to be tested is oriented and at least a further independent variable describing the specific treatment to which the individual has been subjected between at least two different treatments one with the said drug and the second with a placebo or with another known drug, the database comprising also for each individuals one or more dependent variables describing the effects of the said treatments; carrying out an input variable selection; adding to the independent variables selected as input variables the dependent variables describing the effects of the treatments; training and validating an artificial neural network with the selected variables as input variables and with the dependent variables; interrogating the said neural network by inputting the values of the variable describing one of the treatments and obtaining as an output the variable values of the effectiveness of the treatment to which the inputted values of the variable of the treatment correspond according to the trained artificial neural network.

## Description

The invention relates to a clinical trial phase simulation method for drug trials, which method allows to predict the trend of the results of a clinical trial phase of a drug.

Bringing a new drug or biological to market requires that the drug is tested. Testing is carried out by means of clinical trials. Clinical trials comprises three phases. In a first phase the drug is tested on healthy volunteers. If the results are positive, a phase two clinical trial is carried out in which the drug is tested onto a limited number of patients which are affected by the disease against which the drug is thought to be active. In this phase the patients are divided into three groups. The individuals of two of the three groups are treated with placebos of different kind or with existing remedies. The individuals of the resting group are treated with the drug to be tested.

The phase 3 is a clinical test on a very large number of volunteers.

While the first and second phase of the clinical trial have reasonable costs and also can be carried out in reasonable time, the third phase requests very long time and has also very high costs.

For this reason actually the third phase of the clinical trial is often not carried out if the results of the second phase of the clinical trial, i.e. the testing of the drug on a limited number of individuals does not indicate a relevant effectiveness of the drug.

Often the differentiation of the conditions of the individuals treated with the drug and the conditions of the individuals treated with the placebos or other known remedies is not as high as to give sufficient certainty that the drug is really effective and thus that a third phase of the clinical trials would lead to a positive result. Besides the obvious reason that the drug is really non effective, the lacking degree of differentiation of the conditions of the individuals treated with the drug from the condition of the individuals treated with placebos or other known remedies can also be caused by a unlucky choice or distribution of the individuals in the three groups or by the fact that the drug is effective only if the treated individual has certain clinical conditions. So for example the drug may be effective against a certain disease only if the patient is not affected by other dysfunctions such as diabetes or other kind of non normal clinical conditions.

For the above reason there is the danger that a non positive or sufficiently encouraging result of the second phase of the clinical test which is not due to a lacking effectiveness of the tested drug would lead to a decision of abandoning the clinical trials without having carried out the third phase of the clinical test. This would lead to the fact that the drug will not be available on the market. Such a situation is a great damage to the public health and there is a need to provide for a tool which would enable the pharmaceutical industry in better evaluating the results of the previous phases of the clinical test in order not to stop the trial before the testing of the drug has been successfully terminated.

Actually there are known computer aided simulators for clinical trials, for example the simulation method and the simulator proposed by Pharsight Corporation, Mountain View, California, USA. The basic features of the said known simulator is disclosed in " Case Study in the use of Bayesian hierarchical modelling and simulation for design and analysis of a clinical trial, by William R. Gillespie, Bayesian CTS example at FDA/Industry workshop September 2003, which can be also downloaded from the site www.pharsight.com. The method on which the known simulator operates is a well known statistical algorithm known as "Montecarlo Algorithm".

This simulator however is not constructed in order to simulate the results or the trend of the results of a third phase of a clinical result basing on clinical trials carried out on a limited number of individuals as in a typical second phase of the clinical test. Thus the prediction cannot be seen as very reliable. The method furthermore is more oriented on better planning the trials relatively to the kind of individuals and the way the trials has to be carried out in order to have bigger chances of success.

The object of the present invention is to provide for a clinical trial phase simulation method and clinical trial phase simulator for drug tests which can give a high accuracy and reliability of the success of a third phase of the clinical trial basing on the results of the second phase of the clinical trial.

The invention achieves the above mentioned aim by means of a clinical trial phase simulation method comprising the following steps:
a) providing a database comprising for each of a certain number of individuals a predefined number of independent variables each of which corresponds to a certain clinical parameter which parameters are relevant or characteristic for describing or identifying a disease condition against which the drug to be tested is oriented and at least a further independent variable describing the specific treatment to which the individual has been subjected between at least two different treatment one with the drug to be tested and the second treatment with a placebo or with another known drug, the database comprising also for each individuals one or more dependent variables describing the effects of the said treatments observed on the individuals ;
b) carrying out an input variable selection by means of an input variable selection algorithm by feeding the set of independent variables of the database to the said input variable selection algorithm;
c) adding to the independent variables selected as input variables at step b) the one or more dependent variables describing the effects of the treatments;
d) training and validating an autoassociated artificial neural network with the set of selected independent variables as input variables and with the one or more dependent variables;
e) interrogating the trained and validated autoassociated artificial neural network by inputting only the values of the variable describing one of the at least two different treatments to which the individuals has been subjected and obtaining as an output the variable values of the effectiveness of the treatment to which the inputted values of the variable of the treatment correspond according to the trained artificial neural network;
f) repeating step e) for each treatment of the at least two treatments to which the individuals has been subjected;
g) comparing the values of the variables relative to the effectiveness of the different treatments to which the individuals has been subjected which values has been determined at steps e) and f).

A further interrogation step or an alternative interrogation step may be also carried out in which to the trained and validated artificial neural network the values of the dependent variables are inputted, this means the values of the effectiveness of the treatment while the network gives as an output the input variables and particularly the variables relating to the kind of treatment which according to the said network will lead to the inputted effectiveness values.

This further or alternative interrogation step gives the opportunity to have a cross control of the first interrogation results.

Furthermore the artificial neural network will also furnish as a result values of all the other independent variable, which gives a further information, namely the optimum clinical profile of the individuals to be chosen for the trial.

The database is generated usually by the population of individuals and the corresponding clinical, relevant parameters as the parameters describing the treatment and the dependent variables describing the effectiveness of the treatments, which individuals form the group for the second phase of the clinical trial, i.e. the restricted limited group of volunteers.

The population of individuals on which a second phase clinical test is carried out are normally divided into at least two groups. The individuals of one group being treated with the drug to be tested and the other group being treated with a placebo or a known remedy.

Thus the method according to the present invention provides a preventive phase of carrying out a second phase trial of the treatment or drug on a limited number of individuals, while the database used for the simulation steps as described above is generated from the results of the second phase clinical trial.

As it will be shown in the detailed description, tests has demonstrated that evaluating the database of the results of the second phase clinical test by using a known predictive algorithm is not sufficient to lead to a prediction which is non contradictory relating to the above mentioned cross control of the results of the interrogation of the network where first the network is interrogated relatively to the effectiveness of the drug by inputting the variable treatment and at a second stage the network is interrogated relatively to the treatment corresponding to a certain effectiveness by inputting to the network the dependent variables relatively to the effectiveness.

Due to the limited number of cases of the second phase clinical test and due to the fact that the independent variables are often chosen basing on the fact that considering the disease or disturb against which the drug to be tested is oriented, the variables describing the health condition of the candidates are plausibly theoretically relevant, the incapacity of the normal prediction networks or similar systems to furnish useful outputs is probably due to the fact that among the theoretically and/or empirically defined independent variables, i.e. the variables defining the health condition of the individuals there are some relations so that not all variables are independent or have a role for the disease or disturb.

Thus the step of carrying out an input variable selection by means of an appropriate method, particularly an appropriate algorithm is a basic step for the method according to the present invention.

In principle any kind of input variable selection method could be used. There are different known selection method or algorithm.

Best results have nevertheless been achieved by using a particular input variable selection mechanism which is based on a combination of a predictive and an evolutionary algorithm.

This is a so called artificial organism since it combines an artificial neural network operating as a simulation of the human brain or network of neural cells and of an evolutionary algorithm which operate according to the rules of genetic.

A particularly relevant artificial organism consist in a combination of an artificial neural network and a genetic algorithm.

The input selection is carried out as follows: Starting form a database having a certain number of input variables, i.e. of independent variables, and a certain number of records to which the said independent and dependent variables are univocally associated, a first population of artificial neural network is generated by training and testing the artificial neural network with different training and testing databases which databases are formed by distributing the records of the complete database on the said testing and on the said training database the records of each training database may be also varied by eliminating one or more different independent variables. Each of the said artificial neural networks is univocally represented by the specific training database and by the validation score obtained in a testing phase carried out by using the trained artificial neural network on the testing database and by comparing the results outputted by the artificial neural networks with the known results of the testing database.

The said first population of artificial neural networks is then treated as starting population for an evolutionary algorithm, particularly a genetic algorithm which uses as a genome of each artificial neural network the corresponding specific training database. Genetic rules apply to generating new artificial neural networks from the combination of two neural network of the starting population by combining according to the said genetic rules the training databases of the said parent artificial neural networks. For each new generated artificial neural network the score is calculated by carrying out the testing phase and a new generation of artificial neural network is generated in which the training databases have a certain structure.

Repeating the said step some independent variable will fall out from the training database of the artificial neural network and the evolutionary process can be interrupted as soon as a certain score is obtained in the testing phase which is considered satisfying. Thus the artificial organism will give as a result an artificial neural network reaching a satisfying or selected score and having a training database which records comprise only some of the independent variables of the totality of independent variables which were present in the original database.

In principle any auto associated artificial neural network can be used in combination of the said artificial organism.

Best results has been achieved by using a special autoassociated artificial neural network which is known as "New Recirculation Network" which is disclosed in (Buscema & Semeion Group "Reti neurali artificiali e sistemi sociali complessi" Volume 1 chapter 13, Ed. Franco Angeli, 1999). This kind of network better works for carrying out the interrogations at the end of the input selection and network training steps.

Entering more in detail of the above mentioned input variable selection artificial organism, the said organism operates according to the following method steps:
a) providing a database having a certain number of input variables, i.e. of independent variables, and a certain number of records to which the said independent and dependent variables are univocally associated and in which database each record relates to a known clinical or experimental case of a sample population of cases;
b) determining a selection of a reduced number of the certain predetermined number of input variables by means of mathematical tools applied to the database.
c) The said mathematical tools comprising a so called prediction algorithm such as a so called neural network;
d) dividing the database in a training and a testing dataset for training and testing the prediction algorithm;
e) defining two or more different training dataset each one having records with a reduced number of the input variables which reduced number of input variables is obtained by excluding one or more input variables from the originally defined number of input variables, while for each record the reduced number of input variables of the corresponding training set has at least one input variable which is different from the input variables of the reduced number thereof of the other training datasets.
f) training the prediction algorithm with each of the different training sets defined under point e) for generating a first population of different prediction algorithm which are divided into two groups of mother and father prediction algorithms and testing the said prediction algorithms with the associated testing set;
g) calculating a fitness score or prediction accuracy of each father and mother prediction algorithms of the said first population by means of the testing results;
i) providing a so called evolutionary algorithm such as a genetic algorithm and applying the evolutionary algorithm to the first population of mother and father prediction algorithms for achieving new generation of prediction algorithms whose training and testing dataset comprises records whose input variables selections are a combination of the input variable selections of the records of the training and of the testing datasets of the first or previous population of father and mother prediction algorithms according to the rules of the evolutionary algorithm;
j) for each generation of new prediction algorithms representing each new variant selection of input variables, the best prediction algorithm according to the best hypothesis of input variable selection is tested or validated by means of the testing dataset;
k) a fitness score is evaluated and the prediction algorithms representing the selections of input variables which have the best testing performance with the minimum number of input variables utilized are promoted for the processing of new generations.
l) repeating the steps i) to k) until a predetermined fitness score defined as best fit of the prediction algorithm and a minimum number of input variables has been reached
m) defining as the selected relevant input variables the ones related to the input variables of the selection represented by the prediction algorithm having both at least the predetermined fitness score and also the minimum number of selected input variables.

Prediction algorithm are actually known and largely used. A detailed disclosure of the so called Neural Networks is made in "Reti Neurali Artificiali e Sistemi Complessi" Part I Teaoria: fondamenti di Reti Neurali Artificiali Massimo Buscema & Semeion Group Franco Angeli S.r.l. Milano.

Also evolutionary algorithm are known and a more precise description of a so called genetic algorithm is disclosed in Davis Lawrence, Handbook of Genetic Algorithm, Van Nostrand Reinhold New York, 1991 or .

Using the above selection method, there is a risk that the independent variables relating to the treatments to which the two groups of volunteers has been submitted are excluded by the selection algorithm. In this case a first solution is to add the two independent variables relating to the two kinds of treatments to the selected independent variables. This solution does not give sure results since it consist in a kind of forcing the selection algorithm.

A better solution consist in the fact that the algorithm is stopped when a certain degree of fitness score has been reached while the selected independent variables comprise at least one or both the independent variables relating to the treatment.

Although the basic teaching of the invention has been disclosed for simplicity sake in relation to a case where the second phase trial is carried out on two groups by submitting each group to one of two kinds of treatment where one of the treatment is the drug to be tested and the other treatment is a placebo or a known drug, the above method according to the invention can be carried out also considering three or more groups each of the said groups is submitted to a different treatment. In this case one of the treatment is carried out using the drug to be tested and the other treatments uses different kinds of placebos or different kinds of known drugs.

The invention relates also to an apparatus for carrying out a simulation of clinical trials on a large number of patients, i.e. third phase clinical trials, the said apparatus comprising a first virtual network formed by a computing machine and a program for the said computing machine which program forces the apparatus to work as a neural network of the autoassociative kind;

The said network being provided with input channels each one for a variable of a certain number of variables describing relevant clinical data of patients and variables describing the treatment to which a the said certain number of patients has been submitted;

The said network being also provided with a certain number of output channels each one relating to variables describing the effects of the treatments;
the apparatus being also provided with means for reading the variables describing the relevant clinical data of patients and the variables relating to the kind of treatment to which the said patients has been submitted and the corresponding output variables relating to the experimentally ascertained effects of the treatments on the said certain number of patients and for adjusting the network response to the input variables in order to generate the known output variables when the input variable of the said database are fed to the input channels;

Means being provided for manually inputting the variable relating to a kind of the treatment and for reading the corresponding output of the network;

And means being provided for forcing the outputs of the output channels at a value corresponding to a certain effect and reading the corresponding input values of the network.

According to a further improvement, the apparatus is provided further with a program which generates different networks, by eliminating or suppressing different input channels relating to different variables describing the clinical status of the patients in each different network and which program generates combinations of the said different networks by combining the sets of the different active or eliminated or suppressed channels of each pair of networks according to combination rules following the basic biologic gene combination rules between parents;

Means being provided for evaluating the accuracy of prediction of each network

And means being provided for stopping the generation of new networks before the selection mechanism eliminates or suppresses the variables describing the treatments.

Further improvements of the method and of the apparatus according to the invention are subject of the depending claims.

The invention will appear more clearly from the following description of an experimental case and from the accompanying tables and drawings in which:
Figure 1 illustrates the step of defining a simulated second phase trial database starting forma third phase trial database of a trial carried out for a known drug and having known results.
Figure 2 illustrate the steps of the method according to the present invention by means of a block diagram and in relation to a first experiment.
Figure 3 Illustrates the nodes dynamics of the artificial neural network used in for carrying out a first experiment
Figure 4 illustrates the steps of the method according to the present invention by means of a block diagram and in relation to a second experiment.

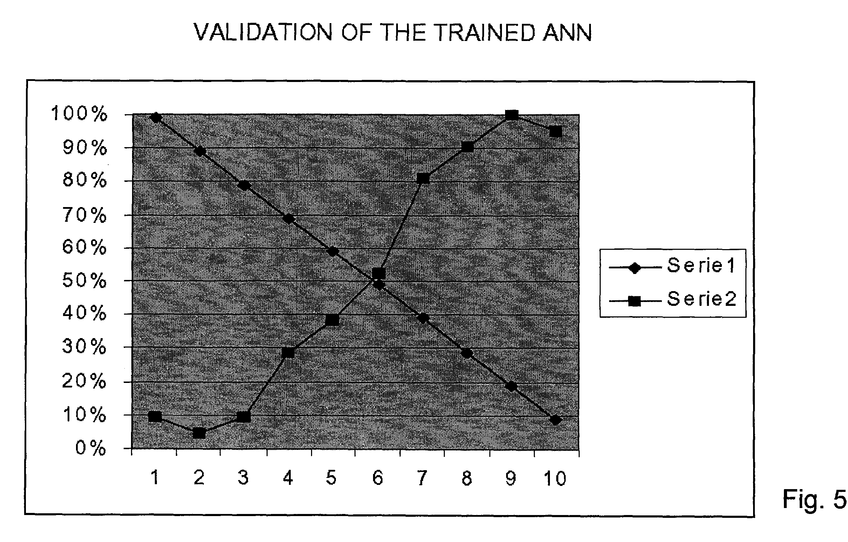

Fig. 5 illustrates a diagram relating to the validation of the trained artificial neural network in the second experiment.

The method according to the present invention is explained in the following by means of two experiments.

The two experiments are carried out using the same starting database which has been created using the database of a third phase trial carried out for a known drug and having given known and certain results. The starting database is created so to simulate a second phase trial database by reducing the number of groups of individuals subjected to different treatments to only two groups of individuals, one group of which was subjected to treatment with the tested drug while the other group of individuals was subjected to treatment with a placebo. Furthermore from each group a reduced number of individuals forming the group is chosen. The choice is also carried out in such a way as to provide trials results which does not lead to a clear indication of effectiveness of the tested drug on the contrary to the results of the third phase trial which has demonstrated that the drug had e clear effectiveness on the disease against which it is directed.

Thus a typical second phase trial situation is constructed according to which the second phase trial gives no clear indications on the effectiveness of the drug tested, while in reality the drug is effective.

The experimental database from which simulated second phase trial database is constructed is the database of the third phase trial relating to the pharmaceutical product named FRILIVER. Detailed information about the trial and the drug are disclosed in the following article "Nutritional Supplementation With Branched-Chain Amino Acids in Advanced Cirrhosis: A Double-Blind, Randomized Trial" of GIULIO MARCHESINI, GIAMPAOLO BIANCHI, MANUELA MERLI, PIERO AMODIO, ARMINE PANELLA, CARMELA LOGUERCIO, FILLIPO ROSSI FANELLI and ROBERTO ABBIATI, carried out for the ITALIAN BCAA STUDY GROUP and published on GASTROENTEROLOGY 2003;124:1792-1801.

The drug has the aim of reducing hospital admission of patients by a nutritional approach which might prevent progressive liver failure, improve nutritional parameters and quality of life. Several clinical parameters of the patients treated were taken into consideration. The said parameters formed part of the independent variables of the database records and will explained in greater detail in the following. Among the independent variables there were three variables indicating three different treatments carried out onto three different groups in which the totality of the patients which attended the trial were divided. Two of the said three groups were treated with a placebo, which was different for each of the two groups and the third group was treated with the drug to be tested.

The dependent variable, namely the results of the trial indicates whether a patient subjected to a treatment has necessitated of an hospital admission after a certain time. This in order to evaluate the effectiveness of the drug.

The complete database had 168 records each belonging to a different patient. Three groups were formed according to the different three treatments provided.

The independent variables were formed by the clinical relevant physiologic parameters of the patients relating to the pathology and to the drug to be tested and are listed in the following table 1.

Table 1 comprises also the indication of the two dependent variables which had to give the measure of the effectiveness of the drug, namely hospital admission and no hospital admission.

| THE 41 INDEPENDENT VARIABLES AND THE TWO DEPENDENT VARIABLES OF THE THIRD PHASE TRIAL | | | |
|---|---|---|---|
| INDEPENDENT VARIABLES | | 23 | GOT |
| 1 | AGE | 24 | FOS ALK |
| 2 | SEX | 25 | QUICK |
| 3 | TREATMENT A | 26 | CARDIAC FREQUENCY |
| 4 | TREATMENT B | 27 | SYSTOLIC ARTERIAL PRESSURE |
| 5 | TREATMENT C | 28 | DIASYSTOLIC ARTERIAL PRESSURE |
| 6 | ETIOLOGY | 29 | REITAN |
| 7 | DURATION OF THE DISEASE | 30 | ASCITES |
| 8 | RIC. OSP AP | 31 | WEIGHT |
| 9 | NI RIC. OSPEDALIERO AP | 32 | HEIGHT |
| 10 | SMOKER | 33 | BMI |
| 11 | SPENOM | 34 | CIRCUMFERENCE OF THE WRIST |
| 12 | Plt<100 | 35 | CIRCUMFERENCE OF THE ARM |
| 13 | CHILD-PUGH | 36 | TSF |
| 14 | RED BLOOD CELL | 37 | BSF |
| 15 | HEMATOCRIT | 38 | SISF |
| 16 | HEMOGLOBIN | 39 | SSSF |
| 17 | LEUKOCYTE | 40 | MAMA |
| 18 | THROMBOCYTE | 41 | MAFA |
| 19 | CREATININE | | |
| 20 | ALBUMIN | | |
| 21 | BILIRIRUBINA TOTALE | | |
| 22 | GTP | | |
| | | | |
| | DEPENDENT VARIABLES | | |
| 1 | HOSPITAL ADMISSION | | |
| 2 | NO HOSPITAL ADMISSION | | |

The three groups of individuals which took part to the trial were formed according to the following table 2

| | | |
|---|---|---|
| GROUP 1 | TREATMENT A | PLACEBO A |
| GROUP 2 | TREATMENT B | PLACEBO B |
| GROUP 3 | TREATMENT C | DRUG TO BE TESTED |

The result of the known third phase clinical trial was that in advanced cirrhosis, long-term nutritional supplementation with the tested drug is useful to prevent progressive hepatic failure and to improve surrogate markers and perceived health status and thus in reducing the need of hospital admission of a patient. This means that relating to the dependent variable "hospital admission" and "no hospital admission", the treatment clearly has shown to reduce cases of hospital admission since in the duration fo the trial patient treated with the tested drug were considerably less subjected to hospital admission than patients treated with the placebos.

So the present known third base trial is an ideal case for testing the effectiveness of the present method since the indications of effectiveness of the drug are clear and unambiguous.

In order to simulate the experimental data of a second phase clinical trial and for sake of clearness, only the two groups relating to a treatment of the placebo and a treatment with the drug to be tested were selected, namely group 2 and 3.

Furthermore the number of records, i.e. of individuals of each group were reduced to a very low number.

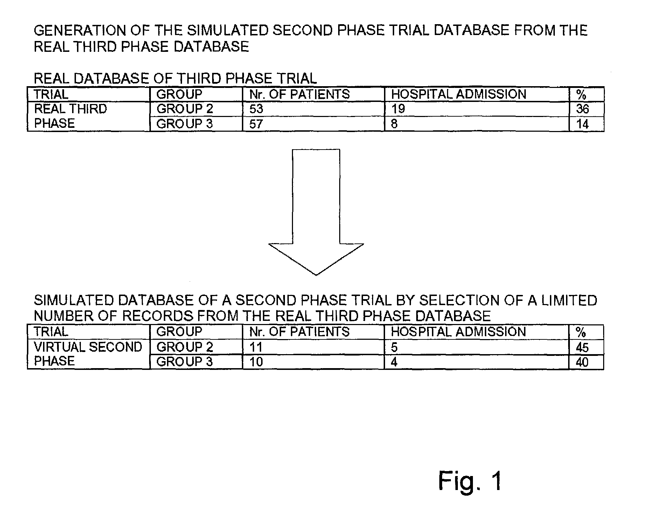

Figure 1 illustrates the step of generating the composition of the experimental data and thus of the database used for carrying out the two experiments of the method according to the present invention.

The composition of the simulated second phase trial database is reported here:

| TRIAL | GROUP | Nr. OF PATIENTS | HOSPITAL ADMISSION | % |
|---|---|---|---|---|
| VIRTUAL SECOND PHASE | GROUP 2 | 11 | 5 | 45 |
| | GROUP 3 | 10 | 4 | 40 |

As it might appear clearly from figure 1 by comparing the virtual database for the second phase trial and the database of the third phase trial carried out in reality, the results of the second phase trial does not give any clear indication on the effectiveness of the drug in reducing the need of hospitalization of the patients, while the result of the third phase trial demonstrates on the contrary that there is an effectiveness of the drug in reducing hospitalization of the patients.

As already disclosed above the invention aims to simulate a third phase trial result basing on the results and the database of the individuals of a second phase trial in order to avoid an abandoning of the trial at the second phase stage if no clear result is given by this second phase trial.

### EXPERIMENT 1

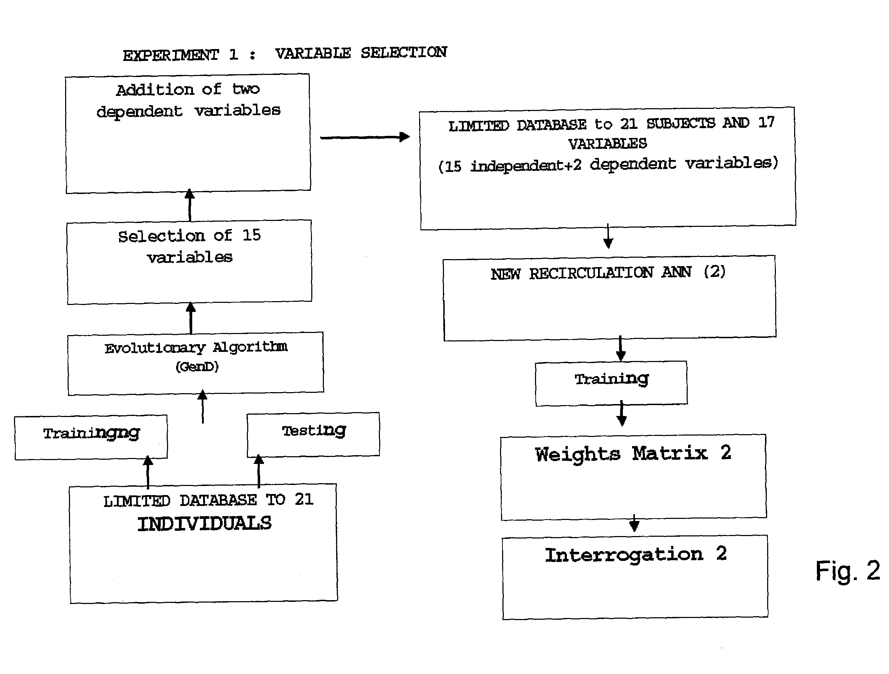

According to the diagram of figure 2, as a first step the above mentioned simulated second phase trial database is used for training and testing a prediction algorithm which might not be limited but which preferably is an artificial neural network. In order to do this the database is divided into a training and a testing database. This step is a usual step which is needed for instructing an artificial neural network or other prediction algorithm. Being the dependent variables of the database known by feeding the independent and dependent variables respectively to the input channels of the prediction algorithm and to the output channels thereof the algorithm "learns", namely adjusts its internal weight matrix in order that the independent variables fed match with the dependent variables within a certain error or precision. Testing consist normally in feeding to the trained algorithm the independent variables and letting the algorithm calculate the dependent variables without forcing the outputs of the prediction algorithm with the said dependent variables with the known values thereof. Afterwards the values of the dependent variables calculated by the prediction algorithm are compared with the known values thereof.

Many kinds of prediction algorithm and among this many kinds of artificial neural networks are available. Among this a particular class of artificial neural networks has given better results and appears to have the best effectiveness and functions in relation of the present problem. This class is the so called class of autoassociative artificial neural networks. In the present experiment a particular autoassociative neural network was used namely a so called New Recirculation Artificial Neural Network which is described with greater detail in Buscema & Semeion Group "Reti neurali artificiali e sistemi sociali complessi" Volume 1 chapter 13, Ed. Franco Angeli, 1999.

Since the selected independent variables are chosen by way of empiric criteria, such as a clinical relevance for the disease, among several variables of the set of independent variables there might be some variables which are false independent variables and which in reality are more or less dependent variables. Due to the fact that the database has a limited number of cases, namely of individuals, this false independent variables may cause the algorithm not to work correctly. Thus as already disclosed above an intermediate step of selecting a reduced number of independent variables is carried out.

This step is also disclosed in figure 2. A certain number of training and testing sets are generated each of which training and testing set is different form the other training and testing set relatively to the number of independent variables and to the kind of the independent variables which are chosen from the totality of independent variables provided in the database.

Thus a population with a certain number of different Artificial neural networks is trained and tested each of which artificial neural networks is univoquely identified by the number and kind of independent variables considered in the training and testing datasets and by the fitness score obtained during the testing step.

This population is used as a starting population for an evolutionary algorithm, particularly a genetic algorithm. The genetic algorithm on which the present experiment was based is the so called GenD algorithm (Genetic Doping Algorithm) which has been developed by Semeion and which is disclosed in greater details in Buscema & Semeion Group "Reti neurali artificiali e sistemi sociali complessi" Volume 1 chapter 21, Ed. Franco Angeli, 1999.

Using the fitness score of each artificial neural network of the starting population of each artificial neural network as a criterion for combining the genoma of two individuals, i.e. of two artificial neural networks of the said starting population, which genoma is formed by the composition of the training and testing database relatively to the number and kind of independent variables considered in the said training an testing database, new generations of artificial neural networks are generated the artificial neural networks of the population of each new generation having training and testing databases comprising different numbers and kinds of independent variables as the parents one and as the complete starting database.

The successive generation of populations is carried out till an artificial neural network is found which fitness score in testing is higher than a predetermined fitness score and which has a minimum number of independent variables in its training database and testing database.

With this step a new database is defined which has a reduced number of selected independent variables in comparison to the starting database coinciding to the complete simulated second phase trial database.

Another criterion has to be considered in this case. Since the problem lies in the fact of determining the effectiveness of the drug in treating a certain disease, the reduced set of selected independent variables should comprise at least one variable related to one of the treatments (treatment b or 2 or treatmente c or 3), or both the variables related to both the treatments to which the two groups of individuals has been subjected in the second pahse trial.

Thus this criterion obliges for stopping the independent variable selection phase not only the fitness score and the minimum number of independent variables in the training and testing database, but also the fact that the reduced database must comprise at least one of the two independent variables describing at least one of the treatments.

In the present experiment 1 as indicated by fig. 2, the independent variable selection phase was stopped when the independent variables for both the treatments were still present among the reduced number of selected variables in the training and testing databases.

The independent variables selection phase was stopped at a generation in which the artificial neural network having the best fitness score had only 15 independent variables selected from the 21 of the original starting database defined as the complete simulated second phase trial database.

To this database the two dependent variables were added thus generating a new staring database for training the artificial neural network. This new reduced staring database comprises 21 individuals and for each individual 15 selected independent variables relating to 15 selected clinical parameters and 2 dependent variables relating to the results "hospitalization" and "no hospitalization".

This new reduced staring database is used for training a New Recirculation artificial network. In training the network a weight matrix for the knots of the network is generated. The network can be than interrogated.

Interrogation takes place as follows. The corresponding Input knots of certain variables either independent or dependent variables are forced to certain values and the values of the other variables either independent and dependent are obtained which according to the artificial neural network and to its weight matrix fits the values inputted for the certain variables.

According to the present invention two kind of interrogation may be relevant.

A first kind comprises to input the values of the independent variables relating to the treatments and then observing the output given by the artificial neural network for the dependent variables.

In the present case this means inputting the value for indication a treatment either B or C and considering the values for the variables "hospitalization" and "no hospitalization" given by the artificial neural network at its outputs.

A cross interrogation may be carried out in which the value for the dependent variable is given to the output of the artificial neural network, forcing the output at these values and considering the response of the artificial neural network for the independent variables relating to the treatment.

The two interrogation give then a clear idea if the results obtained by the artificial neural network are true or false, depending on whether or not the results of the two interrogation are consistent or contradictory.

The following tables 3 to 6 illustrates the results of different interrogations.

In table 3 the artificial neural network was asked to predict the hospitalization or no hospitalization in the case that a patient is subjected to treatment B, namely to the placebo. This is done by imposing to the independent variable Treatment 2 the I value, namely the inputted value 1.

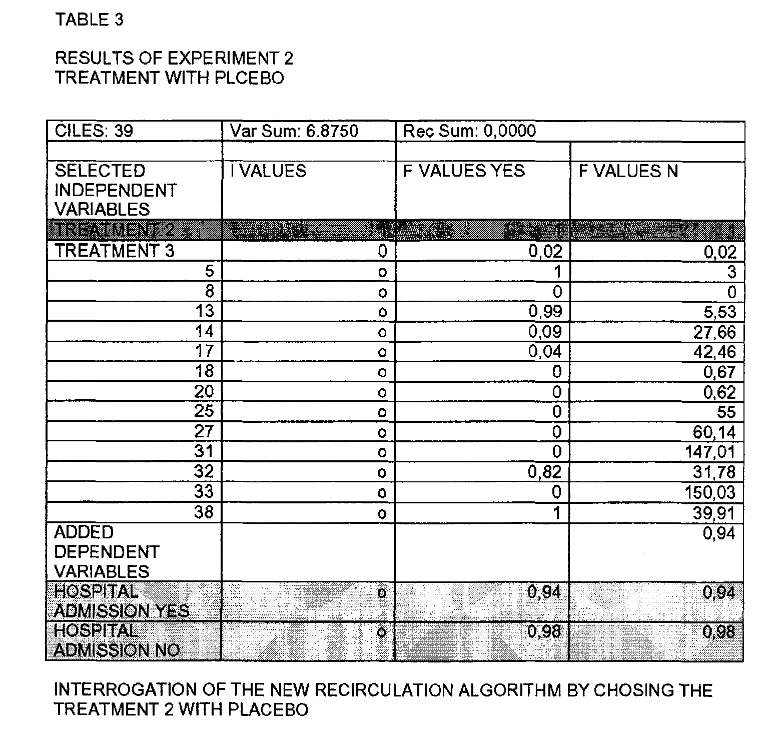

As it appears clearly the result is not clear because both dependent variables "Hospital admission Yes" and "Hospital admission No" have values higher than 0,9, close to 1.

Similar results are obtained if considering the complementary case in which the artificial neural network is asked to predict hospitalization in the case that an individuum is subjected to treatment 3, namely to a treatment with the drug. This result is illustrated in the following table 4. In this case the I value (Input value) for the independent variable Treatment 2 is zero and of the independent variable treatment 3 (treatment with the drug to be tested) is 1.

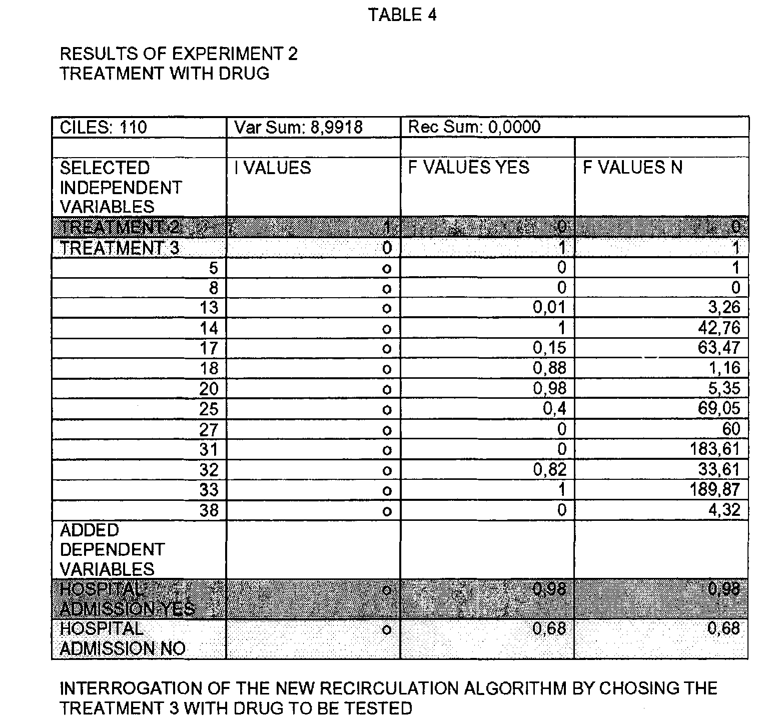

Both the results of the two interrogations give no indication about the fact that the tested drug is effective nor ineffective and both this results are in contrast with the results of the third phase trial which was carried out on the drug FRILIVER ®.

Changing however point o view, positive results could be obtained.

In a third interrogation, the artificial neural network was asked to predict which kind of treatment has to be used for obtaining "no hospital admission".

In this case as it will become clear from table 5, the output of the dependent variable "No hospital admission and "Hospital admission" were forced respectively to 1 and 0 as can be seen in the column relative to the I value (Input value).

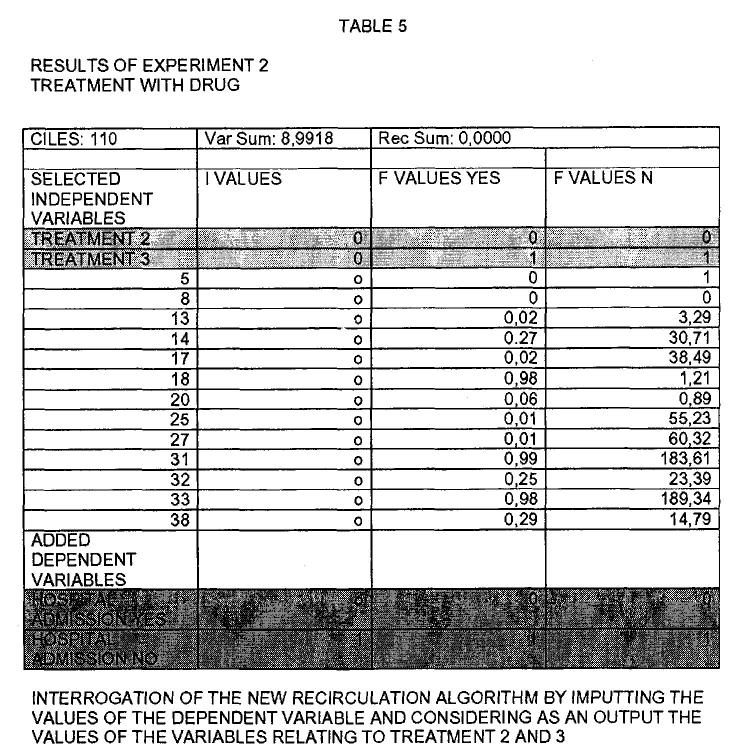

By forcing the values of the dependent variables "Hospital admission NO" and Hospital admission Yes" respectively to 1 and 0 representing the case that no hospital admission is necessary or is occurred, the artificial neural network of experiment 1 sets the values of the independent variables " treatment2" (placebo) and "treatment3" (drug) respectively to 0 and 1, thus indicating that "No hospitalization" occurs when "Treatment3", namely the treatment with the drug is applied. This result is consistent with the result of the third phase trial carried out in reality.

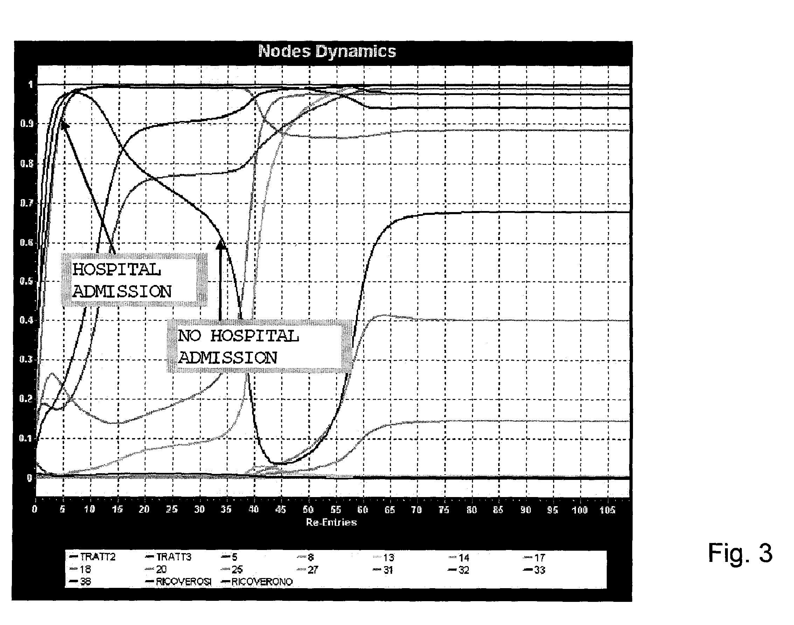

According to figure 3, where the nodes dynamics is illustrated by reporting the nodes values on the y-axis and the repetition cycles on the x-axis, for the nodes related to the different variables, a further indication can be derived from the artificial neural network. In fig. 3 the velocity with which the nodes relating to the dependent variables "Hospital admission Yes" and Hospital Admission NO" are indicated for the interrogation of table 3, where the artificial neural network was requested to determine if hospitalization will be predicted if treatment 2, namely is applied, this means if the patient is treated with the placebo instead than with the drug to be tested. One can see that the node relating to the variable Hospital Admission" yes reaches very rapidly the value close to 1, while the node of the dependent variable corresponding to "No Hospital Admission" has not yet reached the final value after 105 repetition cycles of the algorithm.

Thus although the results of the interrogations of table 3 and 4 did not lead to any indication about the effectiveness, the results of the interrogation according to table 5 and the analysis of the node dynamics are clear indications that there might be a drug effectiveness. This also if the simulated second phase trial did not show results indicationg an effectiveness of the drug.

### EXPERIMET 2

In the second experiment, the same simulated second phase trial database was used and the same criteria and method steps were applied to the said database.

Again a preventive processing phase comprising independent variable selection was applied prior to training, validating and interrogating the artificial neural network.

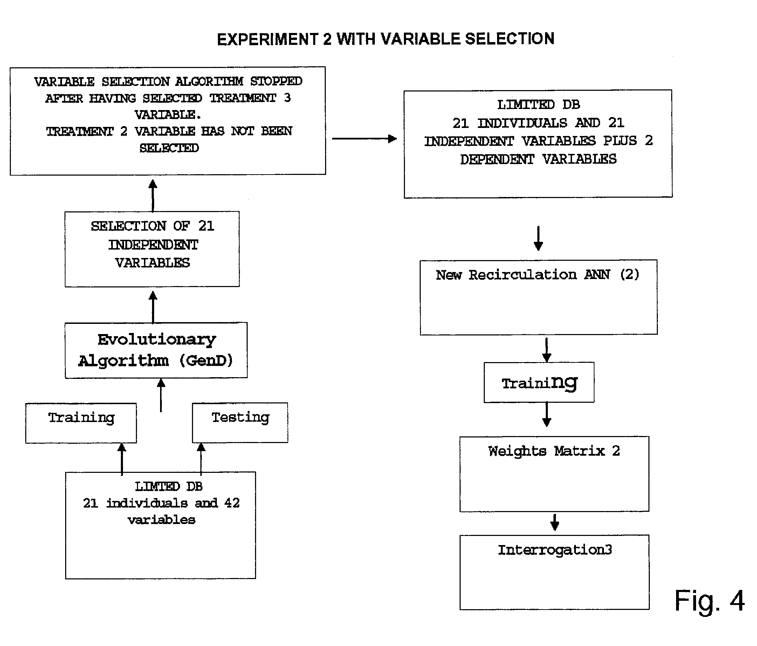

The steps of the method applied in the second experiment are illustrated in the diagram of figure 4. In this second experiment 21 independent variables were selected by means of the preventive processing variable selection phase. Differently form the first experiment, here the variable selection phase was stopped at a stage where among the selected variables only treatment 3 was present, namely the treatment with the drug.

To the 21 selected independent variables gain the two dependent variables were added and a new recirculation algorithm was trained, validated and interrogated.

Table 6 illustrates the validation diagram of the new recirculation algorithm trained with the reduced database after having carried out the independent variable selection.

The interrogation done in table 6 consists in inputting the value 1 for the independent variable describing treatment with the drug, namely "treatment 3" and considering the outputs given by the artificial neural network for the two dependent variables "Hospital admission yes" and "Hospital admission No".

As it will appear clearly from the following, the results indicates a value of zero for the dependent variable "Hospital admission Yes" and a value of 1 for the dependent variable " Hospital admission No", this means that by considering a treatment of a patient with the drug to be tested no hospital admission of the patient is predicted. This result is consistent with the results of the real third phase trial carried out on the drug.

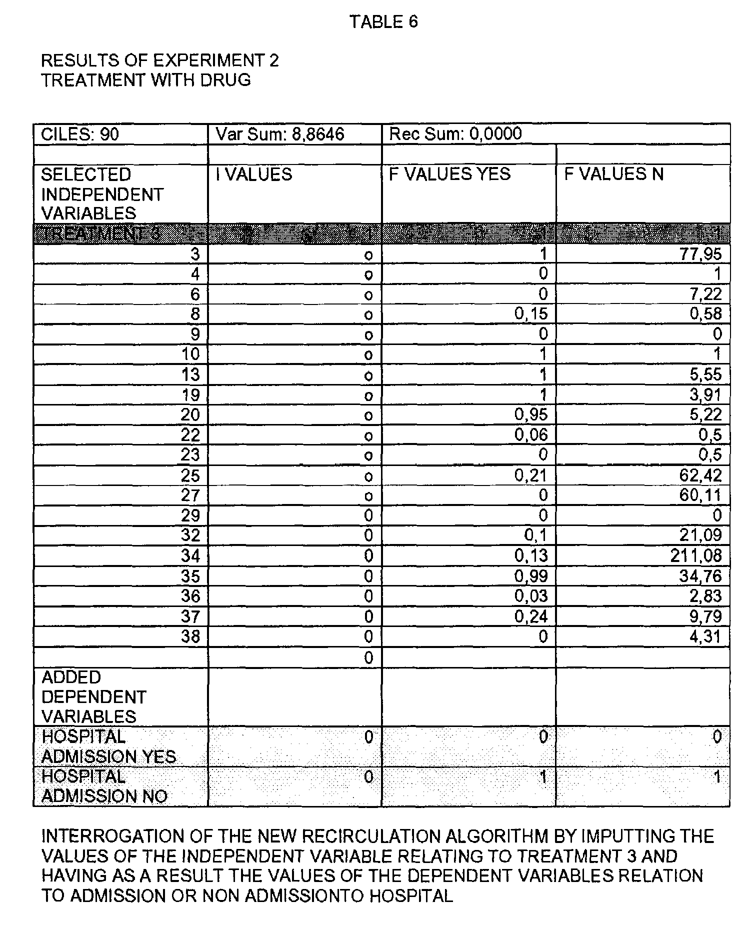

Table 7 relates to an interrogation where the value for the dependent variables are inputted to the artificial neural network. The value 0 is given for the variable "Hospital admission No" and the value 1 is given to the variable " Hospital admission Yes". This is equivalent to query the artificial neural network about the treatment if there is no hospital admission of the patient.

In this case it appears evident that the result is a value 0 for the independent variable "Treatment 3". Thus in this case the algorithm was not able to determine if in case of no hospital admission this situation is due to having treated the patient with treatment 3, namely with the drug.

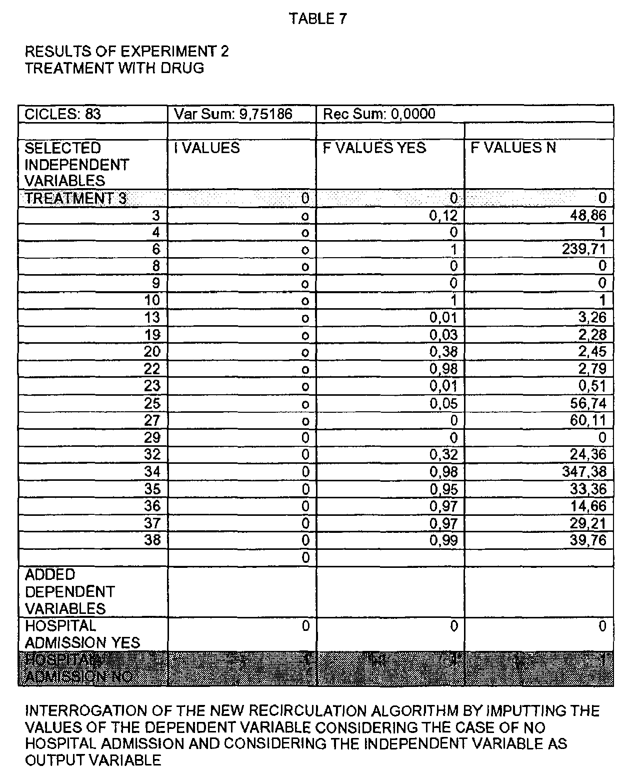

Table 8 depicts the answer of the artificial neural network to the question if in case of no treatment with the drug there will be an hospital admission or not.

In this case the to the variable "treatment 3" the input value of 0 was given, meaning no treatment with the drug.

The variable relating to "Hospital admission Yes" is set to 1 by the algorithm, while the variable relating to "Hospital admission NO" is set to zero or approximately to 0.

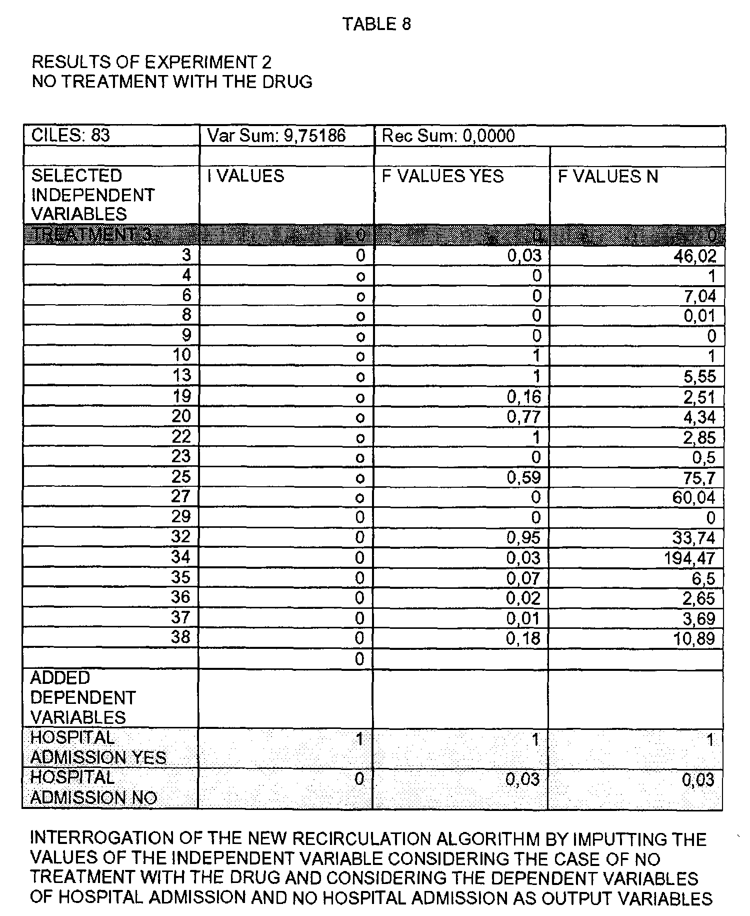

The answer obtained by the artificial algorithm is thus that if no treatment is carried out there will be "hospitalization" of the patient.

The answers to two of three interrogations given by the algorithm according to experiment 2 are thus consistent with the results of the real trial of the drug.

A further important result which is obtained by the method disclosed by means of the two experiments relates to the fact that interrogating the artificial neural network, a certain profile of the values of the other independent variables is obtained.

This independent variables all relates to certain clinical parameters which have a precise physiological meaning, particularly in view of the specific disease for which the drug is tested.

This fact could give information about specific features that the individuals has to have for being optimum individuals for the trial.

Furthermore the said profile of the clinical parameters can give information about the clinical parameters which are really relevant for the disease and also in order to identify special circumstances or combination of values of clinical parameters for which the drug is ineffective or particularly effective, thus constituting a further tool for medical studies of the disease.

From the above it might be appreciated that the method according to the invention is a very useful and low cost tool for simulating third phase trials starting form a second phase trial database. This could help in deciding to carry out a third phase trial or not for a certain drug although the results of the second phase trial did not furnish any indication on the effectiveness of the drug. Thus very high investments can be better checked avoiding loss of money and use of time.

## Claims

1. Clinical trial phase simulation method for drug trials, which method allows to predict the trend of the results of a clinical trial phase of a drug comprising the following steps:
a) providing a database comprising for each of a certain number of individuals a predefined number of independent variables each of which corresponds to a certain clinical parameter which parameters are relevant or characteristic for describing or identifying a disease condition against which the drug to be tested is oriented and at least a further independent variable describing the specific treatment to which the individual has been subjected between at least two different treatment one with the drug to be tested and the second treatment with a placebo or with another known drug, the database comprising also for each individuals one or more dependent variables describing the effects of the said treatments observed on the individuals ;
b) carrying out an input variable selection by means of an input variable selection algorithm by feeding the set of independent variables of the database to the said input variable selection algorithm;
c) adding to the independent variables selected as input variables at step b) the one or more dependent variables describing the effects of the treatments;
d) training and validating an autoassociated artificial neural network with the set of selected independent variables as input variables and with the one or more dependent variables;
e) interrogating the trained and validated autoassociated artificial neural network by inputting only the values of the variable describing one of the at least two different treatments to which the individuals has been subjected and obtaining as an output the variable values of the effectiveness of the treatment to which the inputted values of the variable of the treatment correspond according to the trained artificial neural network;
f) repeating step e) for each treatment of the at least two treatments to which the individuals has been subjected;
g) comparing the values of the variables relative to the effectiveness of the different treatments to which the individuals has been subjected which values has been determined at steps e) and f).

2. A Clinical virtual trial phase according to claim 1, **characterized in that** it comprises a further interrogation step or an alternative interrogation step in which to the trained and validated artificial neural network the values of the dependent variables are inputted, this means the values of the effectiveness of the treatment while the network gives as an output the input variables and particularly the variables relating to the kind of treatment which according to the said network will lead to the inputted effectiveness values.

3. A clinical virtual trial phase according to claims 1 or 2, **characterised in that** it comprises the steps of discriminating similar output values for two different variables by determining the artificial neural network nodes dynamics of the nodes related to the said variables determining which of the two nodes reaches as first the said similar value in a stable manner and setting the output value of the variable corresponding to the said node that has reach as first one the said similar values at the said similar value, while the output value of the other variable/s is set to the value that the corresponding node/s has reached in at the time the other node has reached as the first one the said similar values.

4. A clinical virtual trial phase according to one or more of the preceding claims, **characterized in that** a step of carrying out a variable selection among the variables of the database is carried out.

5. A clinical virtual trial phase according to claim 4, **characterized in that** the variable selection is carried out on the independent variable of the database relating to the clinical parameters relevant for the disease against which the drug is destined.

6. A clinical virtual trial phase according to one or more of the preceding claims 4 to 6, **characterized in that** the independent variable selection step chooses a limited or reduced number of independent variables from the totality of independent variable provided in the original database.

7. A clinical virtual trial phase according to claim 6, **characterised in that** the independent variable selection step is stopped at a independent variable selection stage at which at least one of the variables representing at least one or at least part or all of the treatments provided in the trial is still present among the selected independent variables.

8. A clinical virtual trial phase according to one or more of the preceding claims **characterized in that** the variable selection step is carried out by means of a combination of a predictive algorithm and of an evolutionary algorithm.

9. A clinical virtual trial phase according to claim 8, **characterized in that** the variable selection is carried out by means of the following steps:
providing a starting database having a certain number of input variables, i.e. of independent variables, and a certain number of records to which the said independent and dependent variables are univocally associated;
generating a first population of artificial neural network is generated by training and testing the artificial neural network with different training and testing databases which databases are formed by distributing the records of the starting database on the said testing and on the said training database;
the records of each training database being also varied by eliminating one or more different independent variables in each different training and testing database, so that each of the artificial neural networks generated with a different training and testing database is univocally represented by the specific training database and by the validation or fitness score obtained in testing the said artificial neural network;
the said first population of artificial neural networks being successively treated as starting population for an evolutionary algorithm, particularly a genetic algorithm which uses as a genoma of each artificial neural network the corresponding specific training database;
the said evolutionary algorithm generates a new generation of artificial neural networks comprising a population of artificial neural networks each one of them has been trained and tested by means of a training and testing database which number of variables and which kind of variables are a combination of the number of variables and of the kind of variables of the corresponding training and testing databases of the two parents artificial neural networks;
for each new generated artificial neural network the fitness score is calculated by carrying out the testing phase;
repeating the said steps till a certain fitness score higher than a predetermined lower limit has been reached by a son artificial neural network which also has a minimum number of independent variables selected among the totality of independent variables which were present in the starting database.

10. A clinical virtual trial phase according to claim 9, **characterised in that** the evolutionary algorithm is interrupted when at least one of the independent variables representing at least one or at least part or all of the treatments provided in the trial is still present among the selected independent variables.

11. A clinical virtual trial phase according to claims 9 and 10, **characterised in that** the variable selection is carried out by means of the following method steps:
a) providing a database having a certain number of input variables, i.e. of independent variables, and a certain number of records to which the said independent and dependent variables are univocally associated and in which database each record relates to a known clinical or experimental case of a sample population of cases;
b) determining a selection of a reduced number of the certain predetermined number of input variables by means of mathematical tools applied to the database.
c) The said mathematical tools comprising a so called prediction algorithm such as a so called neural network;
d) dividing the database in a training and a testing dataset for training and testing the prediction algorithm;
e) defining two or more different training dataset each one having records with a reduced number of the input variables which reduced number of input variables is obtained by excluding one or more input variables from the originally defined number of input variables, while for each record the reduced number of input variables of the corresponding training set has at least one input variable which is different from the input variables of the reduced number thereof of the other training datasets.
f) training the prediction algorithm with each of the different training sets defined under point e) for generating a first population of different prediction algorithm which are divided into two groups of mother and father prediction algorithms and testing the said prediction algorithms with the associated testing set;
g) calculating a fitness score or prediction accuracy of each father and mother prediction algorithms of the said first population by means of the testing results;
i) providing a so called evolutionary algorithm such as a genetic algorithm and applying the evolutionary algorithm to the first population of mother and father prediction algorithms for achieving new generation of prediction algorithms whose training and testing dataset comprises records whose input variables selections are a combination of the input variable selections of the records of the training and of the testing datasets of the first or previous population of father and mother prediction algorithms according to the rules of the evolutionary algorithm;
j) for each generation of new prediction algorithms representing each new variant selection of input variables, the best prediction algorithm according to the best hypothesis of input variable selection is tested or validated by means of the testing dataset;
k) a fitness score is evaluated and the prediction algorithms representing the selections of input variables which have the best testing performance with the minimum number of input variables utilized are promoted for the processing of new generations.
l) repeating the steps i) to k) until a predetermined fitness score defined as best fit of the prediction algorithm and a minimum number of input variables has been reached
m) defining as the selected relevant input variables the ones related to the input variables of the selection represented by the prediction algorithm having both at least the predetermined fitness score and also the minimum number of selected input variables.

12. A clinical virtual trial phase according to one or more of the preceding claims, **characterised in that** it comprises a preventive step of carrying out a second phase trial of the treatment or drug on a limited number of individuals, while the database used for the simulation steps as described above is generated from the results of the second phase clinical trial.

13. A clinical virtual trial phase according to one or more of the preceding claims, **characterised in that** it is a simulation of a third phase clinical trial based on the data of a second phase clinical trial.

14. A clinical virtual trial phase according to one or more of the preceding claims **characterised in that** the second phase trial database comprises more than two alternative treatments carried out each on a group of individuals comprising part of the total number of individuals participating to a second phase trial.

15. A clinical virtual trial phase according to one or more of the preceding claims, **characterised in that** an interrogation is carried out by inputting the values of the variables relating to one or more of the treatments provided and by reading the output of one or more of the variables relating to the effects provided.

16. A clinical virtual trial phase according to one or more of the preceding claims **characterized in that** an interrogation is carried out by inputting the values of the variables relating to one or more of the effects provided and by reading the output of one or more of the variables relating to the treatments provided.

17. A clinical virtual trial phase according to one or more of the preceding claims **characterized in that** as a result of an interrogation also the independent variables relating to the relevant clinical parameters or of the other parameters are red in order to provide for a clinical and a anagrafic profile of the best individuals for carrying out a real third phase trial.

18. An apparatus for carrying out a simulated clinical virtual trial phase **characterized in that** the said apparatus comprising a first virtual network formed by a computing machine and a program for the said computing machine which program forces the apparatus to work as a neural network of the autoassociative kind;
The said network being provided with input channels each one for a variable of a certain number of variables describing relevant clinical data of patients and variables describing the treatment to which a the said certain number of patients has been submitted;
The said network being also provided with a certain number of output channels each one relating to variables describing the effects of the treatments;
the apparatus being also provided with means for reading the variables describing the relevant clinical data of patients and the variables relating to the kind of treatment to which the said patients has been submitted and the corresponding output variables relating to the experimentally ascertained effects of the treatments on the said certain number of patients and for adjusting the network response to the input variables in order to generate the known output variables when the input variable of the said database are fed to the input channels;
Means being provided for manually inputting the variable relating to a kind of the treatment and for reading the corresponding output of the network;
And means being provided for forcing the outputs of the output channels at a value corresponding to a certain effect and reading the corresponding input values of the network.

19. An apparatus according to claim 18, **characterized in that** the apparatus is provided further with a program which generates different networks, by eliminating or suppressing different input channels relating to different variables describing the clinical status of the patients in each different network and which program generates combinations of the said different networks by combining the sets of the different active or eliminated or suppressed channels of each pair of networks according to combination rules following the basic biologic gene combination rules between parents;
Means being provided for evaluating the accuracy of prediction of each network
And means being provided for stopping the generation of new networks before the selection mechanism eliminates or suppresses all the variables describing the treatments.

20. An apparatus according to claims 18 or 19, **characterized in that** it comprises:
means for selecting one or more variable among the variables provided in the database;
means for inputting data values for the said one or more selected variable;
means for starting a computing cycle;
means for printing and/or visualizing the values of at least part or all of the other variables computed from the inputted values for one or more selected variables;
means for selection of the variables for which the values has been computed;
means for printing and/or visualizing the computed values of the selected variables among the variables for which the values has been computed.

21. An apparatus according to one or more of the preceding claims 18 to 2o, **characterized in that** it comprises an output for visualizing or printing the nodes dynamics and for univoquely highlighting the nodes corresponding to the selected variables;

22. An apparatus according to claim 21, **characterised in that** it comprises means for determining the computing duration for each node corresponding to a selected variable for reaching a stable computed value.

23. An apparatus according to claim 22, **characterised in that** it comprises means for automatically listing the nodes and the corresponding variables in and ordered manner relatively to the duration of the computation for reaching a stable computed value and means for determining the value of the resting nodes of all or of selected variables at the time one selected variable has reached as the first one a stable computed value.
